# EUROPEAN PATENT SPECIFICATION

(11) **EP 4 334 721 B1**
(45) Date of publication and mention of the grant of the patent: **25.06.2025**
(21) Application number: 22723150.3
(22) Date of filing: 02.05.2022
(51) Int. Cl.: G01N 33/543, B01L 3/00, G01N 33/569

(54) **A NANOFLUIDIC DEVICE FOR RAPID AND MULTIPLEXED SEROLOGICAL ANTIBODY DETECTION**
NANOFLUIDISCHE VORRICHTUNG FÜR SCHNELLE UND MULTIPLEXIERTE SEROLOGISCHE ANTIKÖRPERDETEKTION
DISPOSITIF NANOFLUIDIQUE POUR LA DÉTECTION RAPIDE ET MULTIPLEXÉE D'ANTICORPS SÉROLOGIQUE

(30) Priority: 04.05.2021 EP 21171944
(43) Date of publication of application: 13.03.2024
(73) Proprietor: Paul Scherrer Institut, 5232 Villigen PSI (CH)
(72) Inventor: MORTELMANS, Thomas, 5200 Brugg (CH); EKINCI, Yasin, 8046 Zürich (CH); LI, Dr. Xiaodan, 8032 Zürich (CH)
(74) Representative: Fischer, Michael
(86) International application number: PCT/EP2022/061662
(87) International publication number: WO 2022/233765

(56) References cited:
- EP-A1- 3 778 024
- US-A1- 2020 230 603
- GERSPACH MICHAEL ADRIAN ET AL: "Nanofluidic lab-on-a-chip trapping devices for screening electrostatics in concentration gradients", MICROELECTRONIC ENGINEERING, vol. 175, 1 May 2017 (2017-05-01), NL, pages 17 - 22, XP055849260, ISSN: 0167-9317, DOI: 10.1016/j.mee.2016.12.017

## Description

The present invention relates to a nanofluidic device for geometrical particle immobilization and trapping, in particular for rapid and multiplexed serological antibody detection, preferably including Covid-19 and Influenza A.

Currently, diagnostic testing is mostly performed in a clinical setting, where either a nasal swab or blood sample is taken by a clinician and sent for further testing in a biosafety level 2 facility. Here, trained medical personnel characterizes the biospecimen mostly with respect to either COVID-19 related genetic material or COVID-19 associated antibodies with a PCR or ELISA-test, respectively. In light of this cumbersome procedure, there are clear benefits to the development of a device that is able to provide this information rapidly and cost-effectively without the need for specialized laboratories. Innovative testing solutions are even more needed taking into account that 30.8% of all COVID-19 infections are asymptomatic and previously commercialized lateral flow-based rapid tests have limited sensitivity and only provide binary information (*yes*/*no*) on a possible previous infection.

EP3778024 discloses a lab on a chip device for size selective separation, trapping and manipulation of micro/nanoparticles.

US2020/230603 provides a microfluidic device having a channel with a height at its entrance that is greater than at the exit of the channel.

It is, therefore, the objective of the present invention to provide a nanofluidic device for rapid and multiplexed serological antibody detection that can be manufactured at low cost on a large scale. The device can rapidly probe the concentration of multiple specific antibodies at a certain quantitative resolution and performs better than the state-of-the-art rapid tests in terms of sensitivity and specificity. This objective is achieved according to the present invention by a nanofluidic device for geometrical particle immobilization and trapping, preferably but not limited to, for rapid and multiplexed serological antibody detection, comprising:
a) a channel wall defining by its surrounding structure a channel having a trapping section of decreasing cross-sectional area as seen in a flow direction of a fluid comprising the particles to be trapped dependent on their geometry;
b) said trapping section being penetrated by a plurality of support columns; said columns supporting the channel wall from collapsing;
c) said plurality of column supports are formed as free-standing columns in the material of the channel wall.

This device, therefore, comprises a trapping section that offers a very precise course of the cross-sectional area since the channel walls are supported by these column supports. These support columns may have a circular and/or a rectangular cross-section. In the dependency from the size of the particles, the particles are trapped at the corresponding height of the trapping section. Bigger particles are trapped closer to the fluid inlet, while smaller particles are trapped closer to the fluid outlet of the trapping section.

In order to match the size of the particles to be trapped, it is suitable when the distance of adjacent column supports is chosen to be larger than the size of the largest particles to be trapped. Accordingly, the cross-sectional area of the trapping section may be chosen to be at its inlet larger than the size of the biggest particles to be trapped and at its outlet smaller than the size of the smallest particles to be trapped.

In order to avoid the use of any conveying or pumping means or syringes, the channel and its trapping section can be designed to convey a fluid by capillary force.

In order to have several testing lines for the probe, a plurality of channels and respective trapping sections can be incorporated on the device while using preferably the same fluid inlet. Additionally, to expand on the testing possibilities and enable an additional level of multiplexing, it is possible to use different inlets comprising trapping lines of nanoparticles, for example for performing distinct on-bead antibody testing.

A suitable material type for the channel wall can be achieved when the material of the channel wall is made of thermoplastics, for example, but not limited to poly(methyl methacrylate) (PMMA) and cyclic olefin polymer (COP).

A suitable way for the generation of the column supports can be achieved when the column supports are generated by nano-imprinting of a negative master pattern copy into a free-standing PMMA film, thereby generating a patterned PMMA film. Accordingly, the channel and its trapping section can be generated by UV/O-assisted bonding of the patterned and an unpatterned PMMA film.

In order to properly fulfill its role in supporting the channel and/or the trapping section and/or a capillary force section, the support columns may have a cross-sectional area in the range from 1 µm2 to 2000 µm2, preferably in the range from 10 µm2 to 100 µm2. For example, a support column (can also be designated as a pillar) can be around 20µm wide and around 3.4 µm high at its deepest point in the trapping section. At the shallowest point the pillar can have a height of less than 1 µm, for example 850 nm.

With respect to the possible use cases for the device, each trapping section can be used to perform an on-bead immunoassay. Further, multiple inflows can be provided to perform distinct immunoassays for different antigen targets.

In the investigations, the antigen functionalization of particles of the same size can be different for each inflow. Each trapping section can allow for multiplexed detection of different antibody types by using color multiplexing. Color multiplexing can be done by using secondary antibodies conjugated with fluorescent dyes and have different antigen targets.

Alternatively, multiplexing can be performed by beads of similar geometry, but different color. Thus, the color of the trapped beads can be indicative of its antigen functionalization.

Devices for separation and sorting of micron- and nano-sized particles using geometrical immobilization are crucial in a variety of fields in addition to on-chip immuno-assays. These include, but are not limited to: diagnostics, chemical and biological analysis and environmental assessments.

Furthermore, the "particle" can be replaced with a variety of biological entities where geometrical immobilization is of importance. These biological entities can be viruses, cellular organelles, bacteria and human cells. Of course, the device's trapping geometry can be adapted for the required application.

Preferred embodiments of the present invention are hereinafter described in more detail with reference to the attached drawings, which depict in:
- Figure 1: schematically a fabrication process flow for the 3D thermoplastic nanofluidic device with PMMA being used as an example.
- Figure 2: schematically the fabricated nanofluidic 3D PMMA device;

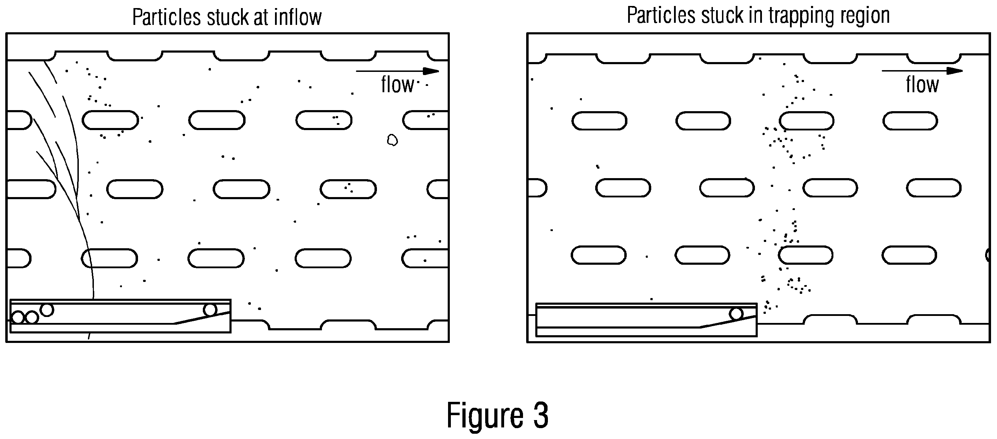

- Figure 3: schematically the influence of PVA on the non-specific binding of streptavidin-coated 1 µm nanoparticles 1 in a 3D nanofluidic device with supporting lines. Left: Sticking of 1 µm nanoparticles to non-functionalized PMMA, Right: Trapping of 1 µm nanoparticles in a functionalized device; The scale bars represent a 100 µm length;
- Figure 4: schematically the filling of the capillary pump and connecting wedge region of devices with and without PVA functionalization, (left) ATTO 448 filled section of the capillary pump and connecting wedge with associated filling fronts and (right) volume over time being loaded into the capillary pump section;
- Figure 5: Light microscopy images evidencing the role of supporting pillars for successful uncollapsed bonding in a 3D nanofluidic device with an outflow of 500 nm, (left) light microscopy image of the collapsed channel due to the absence of supporting pillars (middle) an uncollapsed channel containing supporting pillars to ensure proper separation of both PMMA layers and (right) Zoomed-in light microscopy image of the middle section showing the expected blazed grating-like interference pattern of a bonded device;
- Figure 6: On-chip COVID-19 and Influenza A antibody immune-assay;
- Figure 7: Conceptually illustrating the on-chip multiplexing concept to simultaneously detect IgG and IgM antibodies and disease multiplexing;
- Figure 8: Towards disease multiplexing: saturating biotin-binding pockets of streptavidin; and

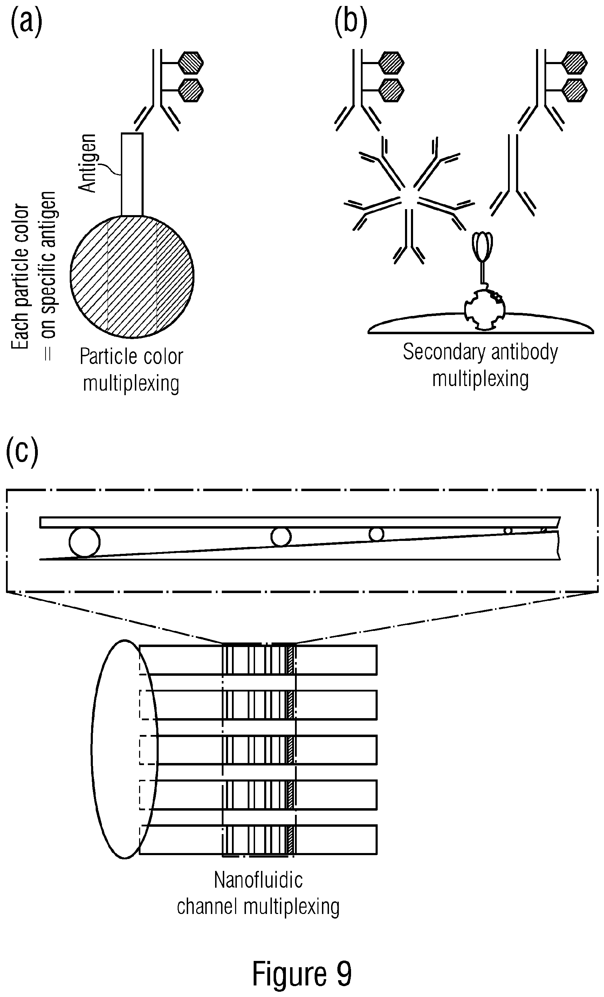

- Figure 9: Various possibilities for multiplexed testing using the nanofluidic 3D device; Particle color multiplexing allows to fluorescently distinguish particles of the same geometry but with different antigens; secondary antibody detection allows for the characterization of different antibodies; nanofluidic channel multiplexing allows to test for specific diseases inside each channel.

In the present invention, a novel 3D thermoplastic-based nanofluidic device capable of geometrical particle immobilization and its nanofabrication are disclosed. Further, the geometry dependent particle trapping sensing potential is shown to perform on-chip serological assays for anti-spike (COVID-19) IgG while providing semi-quantitative data with a high degree of sensitivity and specificity. The device/device is based on fluorescence measurements at predetermined locations, allowing for high sensitivity as well as measurement deportability, in contrast to other bead or microfluidic-based techniques. The device's versatility is shown by performing simultaneous on-bead testing of different antibody sub-types as well as concurrent on-chip detection of COVID-19 and Influenza A antibodies. As both have similar symptoms, this kind of differential testing would allow the treating physician to provide the correct diagnosis and treatment rapidly and accurately.

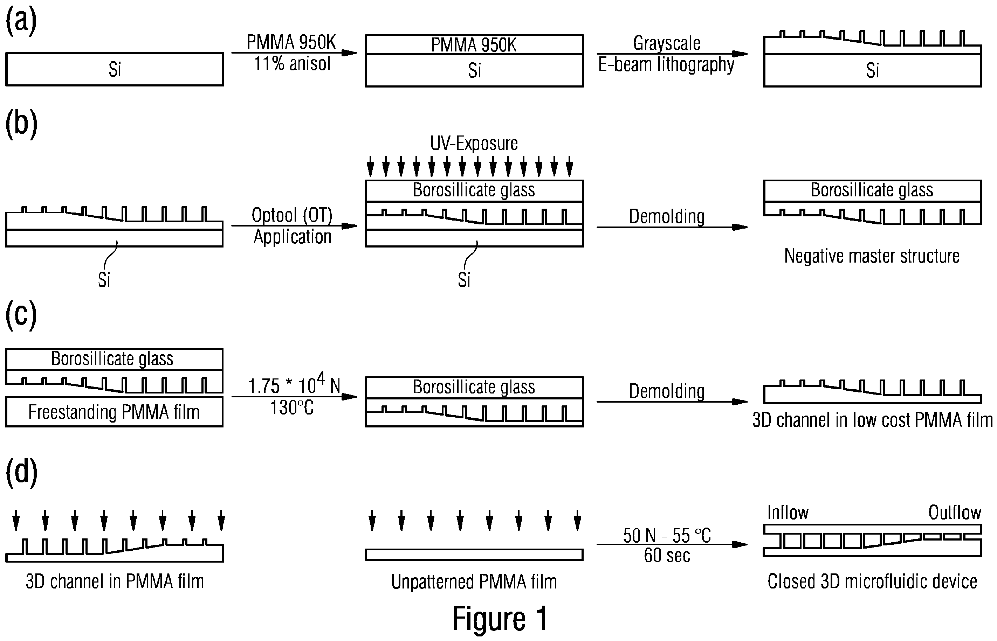

The active region of the nanofluidic device necessitates nanoscale topography control on a millimeter length scale and simultaneous integration of various micro and nanofluidic components. To this extent, high precision gray-scale e-beam lithography (g-EBL) is used to pattern the nanofluidic device following a delayed development process. This high-resolution structure was subsequently replica molded to obtain a negative polymeric stamp, enabling cost-effective upscaling of fabrication through nano-imprint lithography (see Figure 1). Alternatively, the patterns can be realized using alternative fabrication methods such as injection molding or photolithography.

Figure 1 shows a fabrication process flow for a 3D thermoplastic nanofluidic device in PMMA. Fig. 1(a) represents the fabrication of the nanofluidic device in e-beam resist by exposing the material with a spatially dose-modulated electron beam and development in a temperature-controlled developer. Figure 1(b) shows the high-resolution replication of the 3D e-beam pattern by using a commercially available and UV-crosslinkable polymer with inherent anti-adhesion properties. Figure 1(c) illustrates the nano-imprinting of the negative master copy into a free-standing, low-cost PMMA film and Figure 1(d) represents the UV/O-assisted bonding of a patterned and unpatterned PMMA film. The shown fabrication route can be adapted to a large variety of thermoplastic materials and is not limited to PMMA.

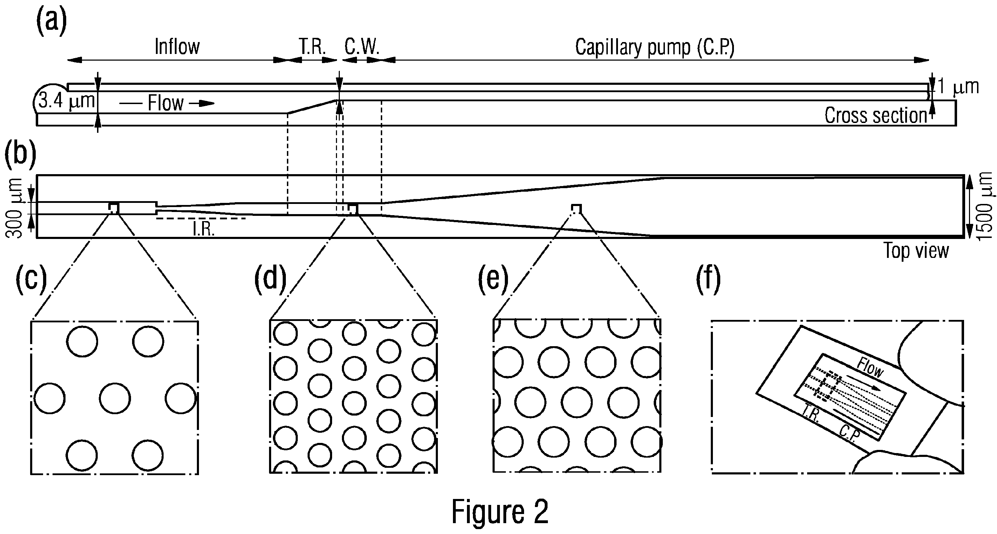

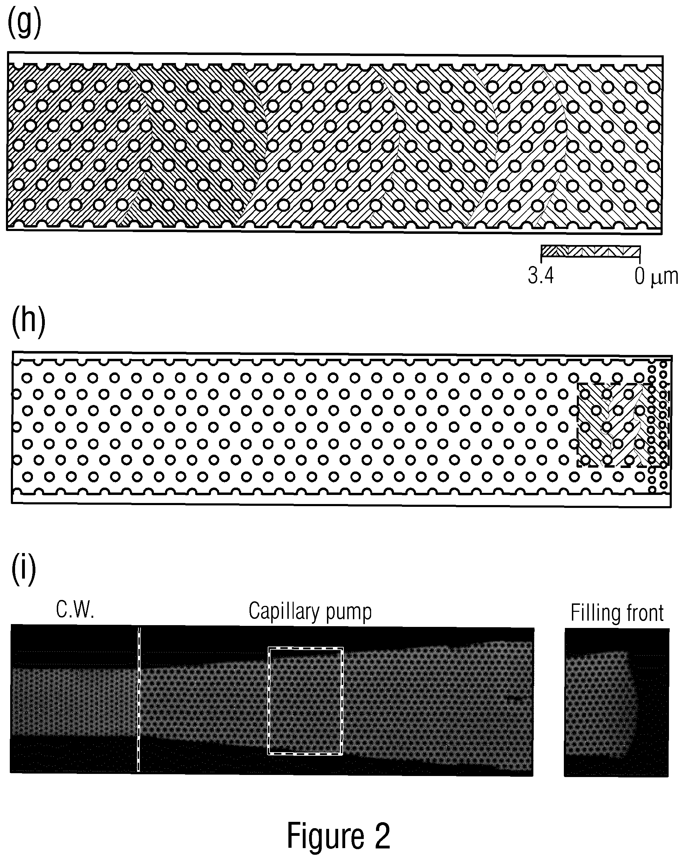

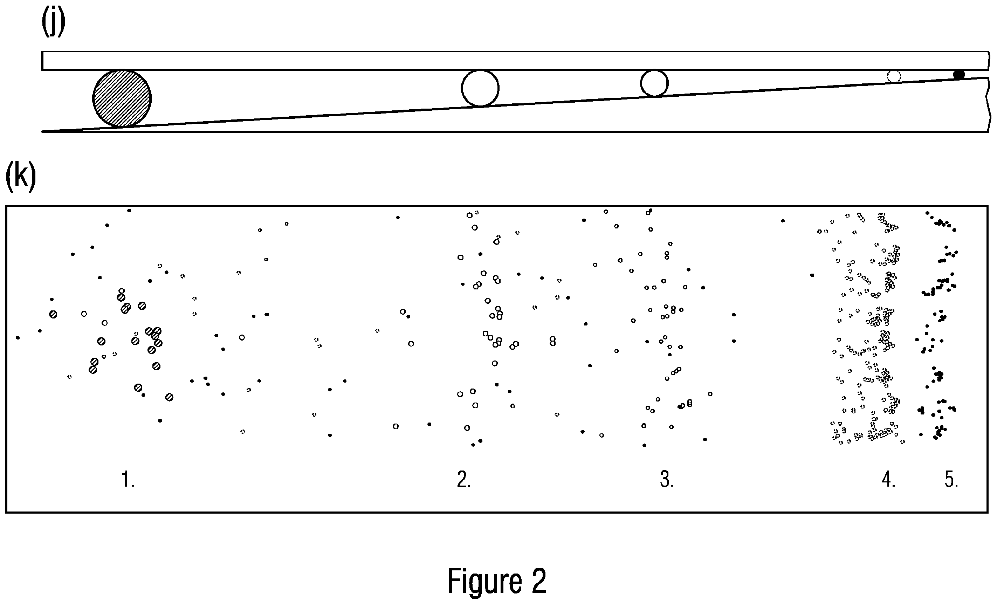

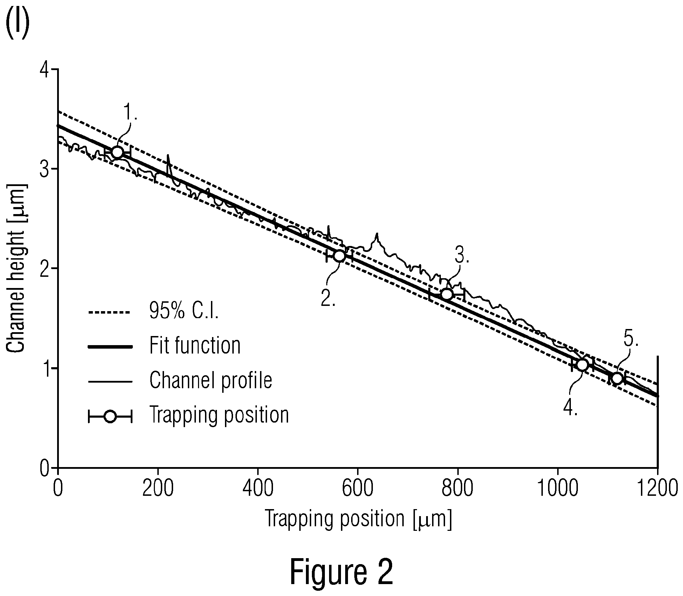

As shown in Figure 2, the fabricated nanofluidic device is designed to contain only passive capillary microfluidic elements to control the fluid flow without the need for external loading mechanisms, such as syringe or pressure pumps. Figure 2(a) shows a cross-sectional view of the changing height profile inside the device. Figure 2(b) shows the SEM top-view image of the device, with the inflow resistor (I.R.) marked by a white dashed line. Figures 2(c), (d) and (f) show SEM images of supporting pillars with a diameter of 20 µm (c,f) and 15 µm (e) in the different regions of the device. Figure 2(f) shows a photograph of the 3D PMMA nanofluidic device showing the narrower inflow and broader capillary pump region. In this specific example, each device contains three channels that have the same geometrical layout to allow for multiple measurements of the same sample on one device. However, it is possible to combine channels with different geometrical layouts to enable trapping of a larger range of particle sizes and increases the device's multiplexing potential. Figure 2(g) is a 2D topographical confocal laser scanning image of the trapping region showing the decreasing channel height over the channel length. Figure 2(h) is an SEM image of the trapping region at a 30° tilt-angle with a light microscopy image of a bonded device inset at the transition between the trapping and connecting region.

Figure 2(i) represents the filling of the connecting wedge and capillary pump region with ATTO488 and the associated filling front, its location indicated by the white dashed line. Figure 2(j) is a cross-sectional sketch illustrating the immobilization of calibration grade particles of different geometries and fluorescence spectra in Figure 2(k) which is a fluorescent micrograph of trapped fluorescent polystyrene calibration grade particles. Figure 2(l) is a plot correlating the mean trapping position with the mean particle size. The error bars represent the standard deviation of the trapping position. The dotted lines show the 95% confidence interval of the linear regression fit. All the scale bars present in Figure 2 represent a 50 µm length.

In this specific example, the device comprises an inflow region of three parallel 300 µm wide inflow channels which is designed for droplet application. Here, each channel passively aspirates the liquid through capillary action (Fig. 2f; left), which is furthered by the presence of 20 µm pillars with a pitch of 40 µm (Fig. 2c). The inflow region is succeeded by a channel narrowing to 100 µm. This width is then kept constant for a length of 500 µm, after which it slowly returns to 300 µm (Fig. 2b - I.R.). The narrower section is designed as an inflow resistor (IR) and retention valve-like structure to minimize premature drying of the liquid inside the nanofluidic device. The IR is followed by the trapping region (TR), where a wedge structure linearly decreases the channel height from 3.4 to 0.8 µm over a distance of 1.2 mm. After the TR, the diameter of the supporting pillars is reduced to 15 µm with a pitch of 22.5 µm (Fig. 2d). The channel height is kept constant at 0.8 µm for 100 µm before a secondary wedge increases the total height to 1 µm over 1 mm. This connecting wedge CW couples the active device region to a capillary pump containing 20 µm pillars with a 30 µm pitch (Fig. 2e).

The resulting topography variation in the trapping region TR is linear in the center of the structure but differs when compared to the channel's edges (Fig. 2g). More specifically, the channel's center is consistently deeper, and this discrepancy is even more pronounced at larger overall pattern depths (Fig 2g; left). Systematic investigation of the various fabrication steps has shown that this effect can be attributed to the difficulty of correcting for the electron proximity effect in three dimensions. As a result, the electron energy deposition in deeper resist regions is less accurately described, giving rise to more prominent stitching lines (Fig. 2g and h; left) in comparison to the shallower sections of the device trapping region (Fig. 2e, Fig. 2h right).

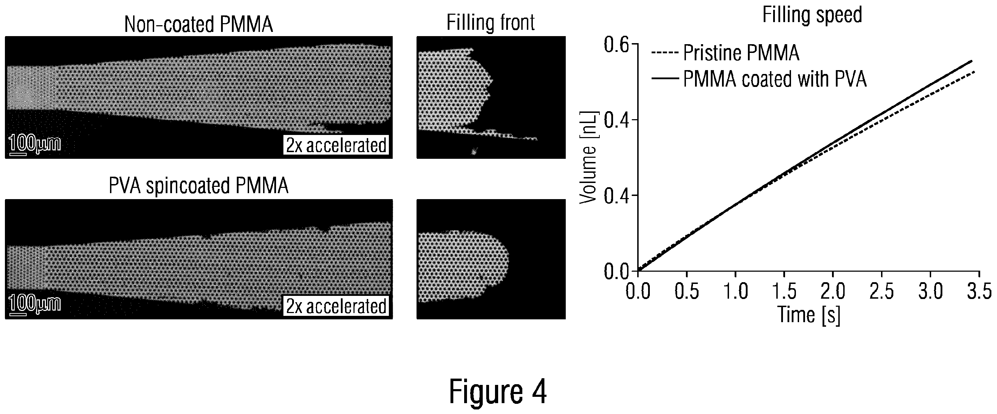

Before proceeding with the particle trapping experiments, the devices were functionalized with poly(vinyl alcohol) (PVA). This blocks non-specific binding sites on the PMMA and allows for more controlled surface wetting. The latter is evidenced by the absence of corner flow in the capillary pump region and improvement of the filling front (Fig 2i; Fig. 4;).

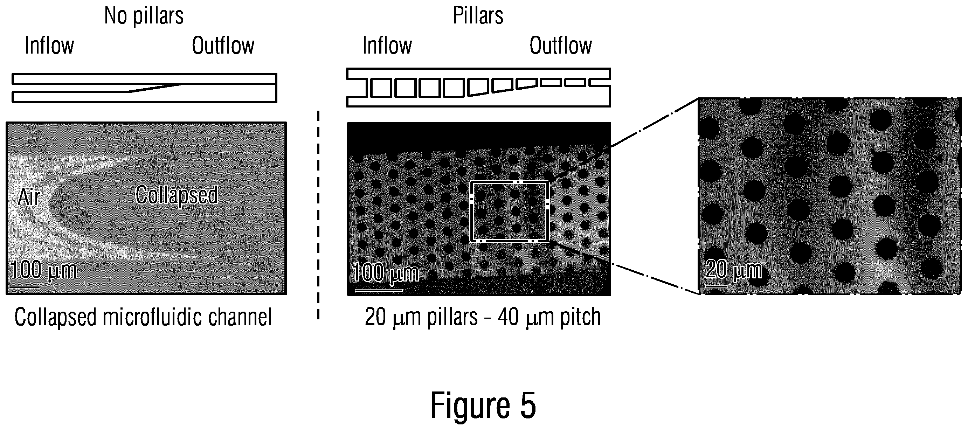

The particle trapping capability of the device was demonstrated by the successful size separation of 5 different fluorescent polystyrene calibration grade particles in the TR (Fig. 2 j-k). The relationship between the particle trapping position and its nominal diameter is highly linear (R² = 0.998; Fig 2l) and can be used to cost-effectively determine nanoparticle sizes (see Table 1). By extrapolation from the linear fit (Fig. 1j), the minimal TR channel height is 720 nm, indicating a reduction of roughly 45 nm when compared to the unbonded height of 765 nm. This nanoscale height loss can be attributed to the surface selective bonding process and also demonstrates the necessity of supporting pillars inside the nanofluidic channel (Fig 1h, inset; Fig. 5).

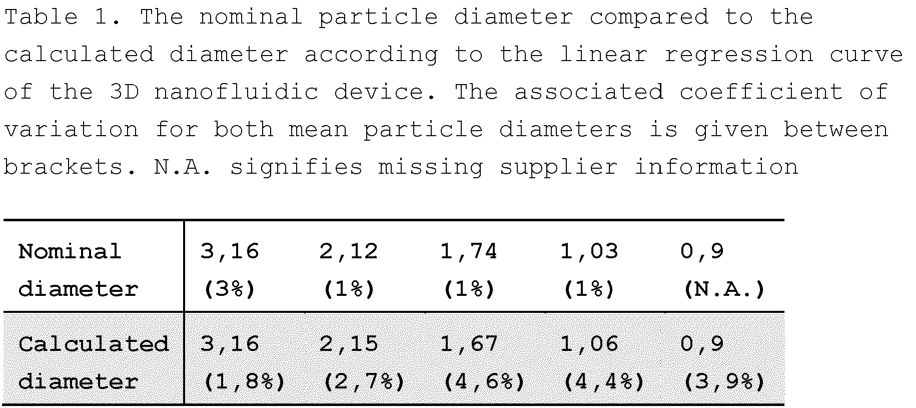

The primary role of the human immune system is to fight infection. To do so, both innate and adaptive mechanisms are in place to efficiently detect and eliminate foreign bodies. Whereas the innate immune system is mostly governed by broad genetically hard-wired responses, the adaptive immune system requires growth and rearrangement of gene elements to produce antibodies that specifically bind to invading antigens. The most commonly found antibody type in human serum is Immunoglobulin G (IgG), making it a valuable target for immune status characterization.

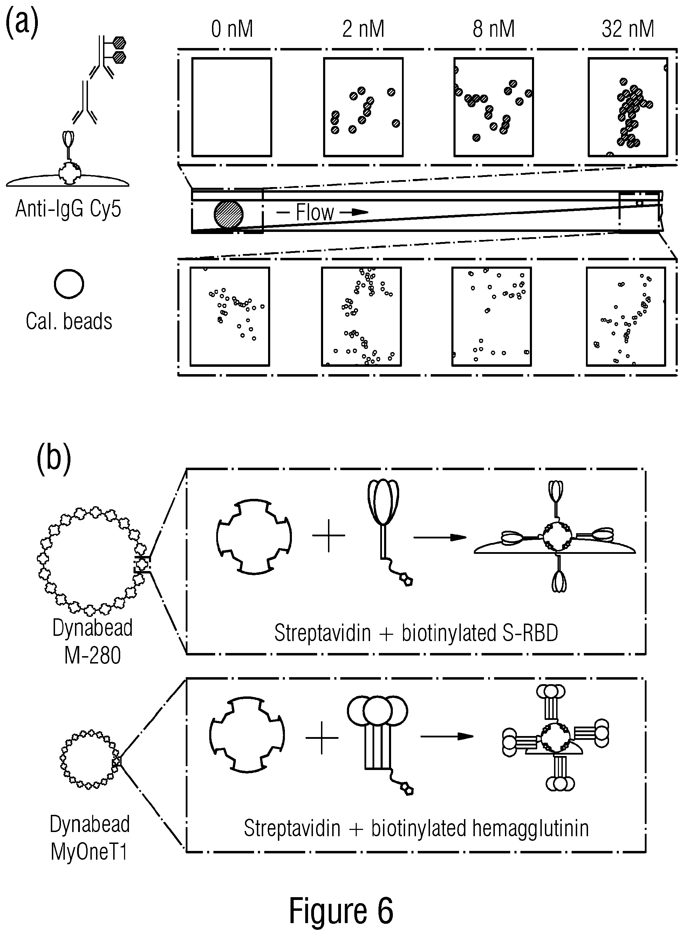

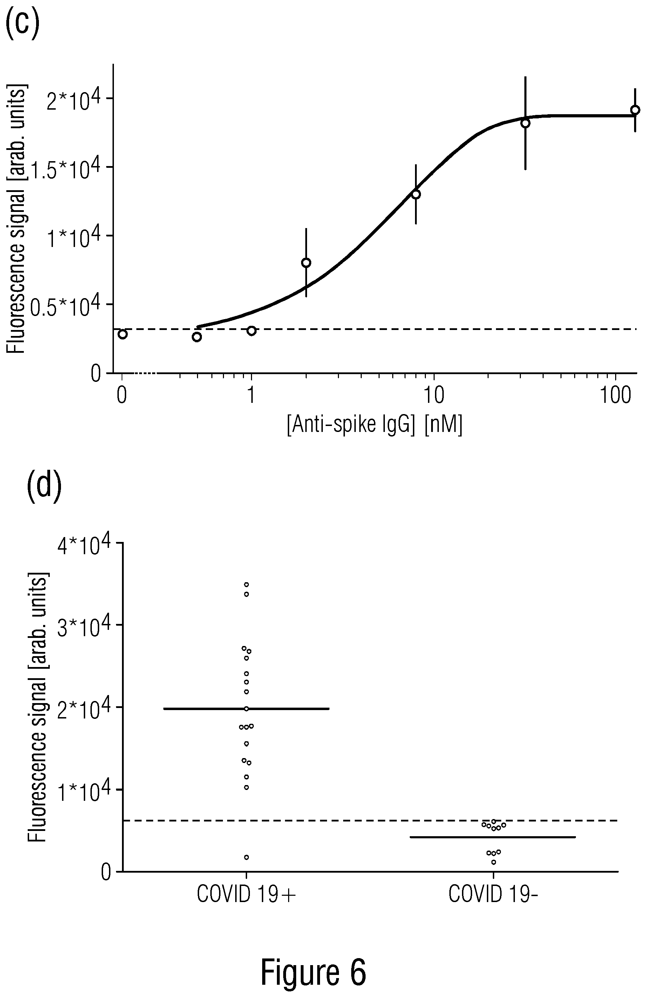

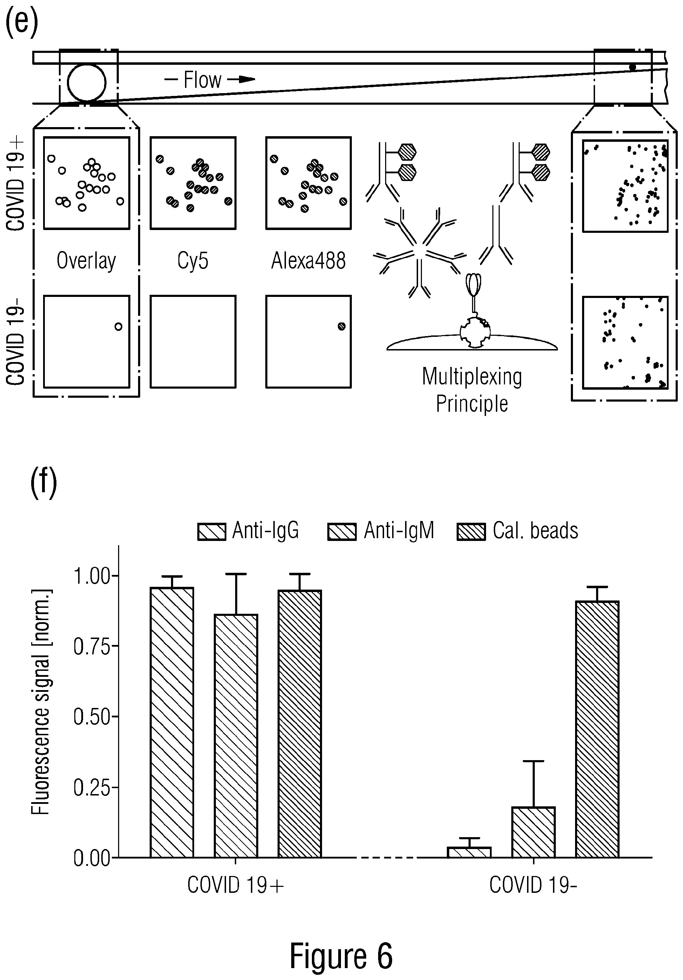

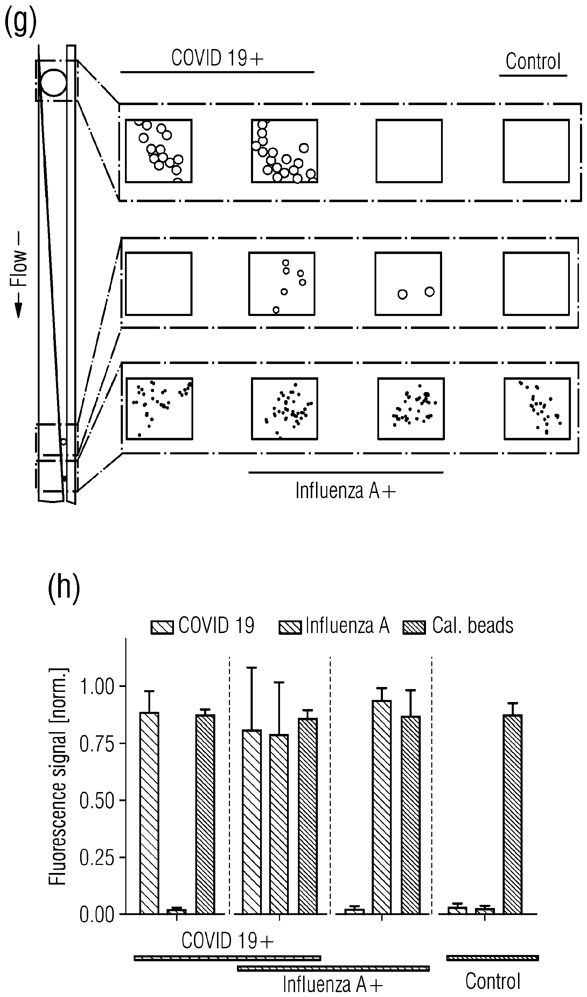

Figure 6 shows an On-chip COVID-19 and Influenza A antibody immunoassay. Figure 6(a) shows Top: Fluorescence images of trapped 2.8 µm nanoparticles mixed with a given concentration of anti-spike antibodies in human serum. Middle: cross-section of the trapping region. Bottom: Trapped 0.9 µm flow control beads. Figure 6(b) shows the functionalization of streptavidin-coated 2.8 µm nanoparticles the receptor-binding domain (RBD) of the spike protein. For simplicity, the entire trimeric protein is drawn with a single biotin moiety. The smaller 1 µm nanoparticles beads (bottom) are similarly functionalized with a biotinylated hemagglutinin protein. Figure 6(c) represents quantification of anti-human IgG Cy5 fluorescent signal on trapped 2.8 µm nanoparticles for a given anti-spike IgG concentration. The dashed line represents the estimated LOD. Figure 6(d) is the validation of on-chip anti-spike IgG immunoassay using COVID-19 PCR positive and negative patient samples. Each data point represents the mean signal of each patient. The dashed line graphically indicates the cut-off value separating negative from positive values. Figure 6(e) shows (Top) the schematic of a color multiplexed IgM and IgG on-bead immunoassay and (Bottom) the resulting fluorescent signal from the on-chip immunoassay for a COVID 19+ and - patient. Figure 6(f) represents the normalized signal of anti-human IgG cy5, anti-human IgM Alexa 488 and calibration bead fluorescent signal.

Figure 6(g) shows (Left) a schematic of a multiplexed on-chip immunoassay for simultaneous detection of anti-spike (COVID-19) and anti-hemagglutinin H1N1 (Influenza A) antibodies and (Right) measured fluorescent signals of trapped particles of different geometries and different antigen functionalities.

Figure 6(h) is a normalized anti-rabbit Cy3 signal at the trapping lines for 2.8 and 1 µm nanoparticles, testing for the presence of COVID-19 and Influenza A antibodies, respectively.

The surface trimeric spike (S) protein, which plays a vital role in facilitating the entry of the Sars-CoV-2 into human cells, is one of the majorantigens associated IgG antibodiesThe receptor binding domain (RBD) of the S-protein is an immunodominant target for COVID-19 antibodies. To enable their on-chip detection, the high-affinity interaction between streptavidin and biotin is used to couple biotinylated RBD of the COVID-19 S-protein to the surface of 2.8 µm streptavidin paramagnetic beads (Fig 6b; Top).

Once functionalized, the beads were mixed with human serum. If the patient was previously exposed to SARS-CoV-2, the serum will contain antibodies with a specific antigen-binding site for S-RBD and bind to the beads. The latter can be fluorescently visualized by conventional immunostaining procedures. In our case, a donkey originating antibody conjugated with a far-red fluorescent dye (Cy5) was used that specifically binds to human IgG (Fig. 6a left). To benchmark the serological immunoassay, the lowest detectable concentration of a purified humanized anti-S-RBD IgG antibody in human serum was determined. To evaluate the nanofluidic device function, non-functionalized 0.9 µm calibration-grade particles were added to the suspension acting as a flow control before applying the particle mixture onto the device's inflow region.

The corresponding fluorescent signal of the trapped beads was computationally analyzed, and the limit-of-detection (LOD) was estimated to be between 1 and 2 nM (Fig. 6c). This is comparable to other microfluidic-based, state-of-the-art techniques that require more sample processing steps and significantly more advanced infrastructure. In recent studies, the physiologically relevant concentration of this specific type of anti-spike IgG antibody in COVID-19 infected people has been shown to be in the range of 9.6 - 28,880 nM. This shows that the developed on-bead and on-chip nanofluidic device operates well within the physiologically relevant concentrations.

Additionally, the assay was validated using a patient serum set containing both PCR positive (n = 19) and PCR negative (n = 10) patients (Fig. 6d). From the 29 different samples, 28 could be identified correctly according to infection status, with one false negative result. This corresponds to a test sensitivity of 94.7% and a specificity of 100%. It should be mentioned that the false negative was retested and showed a signal above the threshold value. These findings evidence the applicability of the assay in a real-world setting for the IgG serological characterization of suspected COVID-19 patients.

Even though IgG is the dominant immunoglobulin sub-type in human serum, it is mostly associated with late-stage and memory-related immune responses. In the case of COVID-19, they typically occur 7 days after infection. This hampers the applicability of IgG-specific antigen assays for early-stage disease detection. However, other antibody sub-types, such as anti-S RBD Immunoglobulin M (IgM), can be already found in human serum 4 days post infection. Hence, the on-bead and on-chip assay was further developed to include color-multiplexing for simultaneous detection of both antibody subtypes (Fig. 6g). More specifically, the immunostaining step contained anti-human IgM antibodies conjugated with a green fluorescent dye (Alexa 488) as well as anti-human IgG antibodies conjugated with a far red fluorescent dye (Cy5) (Fig. 6h).

The anti-IgG fluorescent signal of the COVID 19+ patient was 25.5 times greater than that of the negative control. Similarly, the anti-IgM signal was 4.9 times greater. This difference between IgG and IgM is to be expected, given that the investigated COVID-19+ serum was taken 33 days after symptom onset. As is well known, IgM antibody levels start to decline roughly 21 days after infection, explaining the findings. Nevertheless, this experiment shows the potential of color multiplexing to semi-quantitatively monitor the anti-S-RBD IgG and IgM of suspected COVID-19 infected people.

Furthermore, it is of interest to perform simultaneous differential testing for the presence of antibodies against diseases with similar symptoms. In the case of COVID-19, one of the most symptomatically similar and prevalent viral infections is Influenza A. The latter has caused several widespread epidemics and it is one of the major targets of yearly vaccination campaigns. To demonstrate the on-chip multiplexing of disease-specific antibodies, smaller 1 µm paramagnetic streptavidin-coated beads were labeled with biotinylated hemagglutinin, an immunodominant Influenza A-associated protein (Fig 6a; bottom).

A particle suspension of 1 µm and 2.8 µm biofunctionalized beads was mixed with various combinations of purified polyclonal rabbit antibodies, targeting either the S-RBD or hemagglutinin protein (Fig 6e). The beads were subsequently immunostained with a Cy3 anti-rabbit antibody. The obtained signal at their trapping positions is shown to correlate very well with the presence or absence of the relevant antibodies for either S-RBD (COVID-19) or hemagglutinin (Influenza A), respectively (Fig. 6f). This proof-of-principle experiment further emphasizes the versatility of the present 3D nanofluidic device within the framework of serological multiplexed immunoassays.

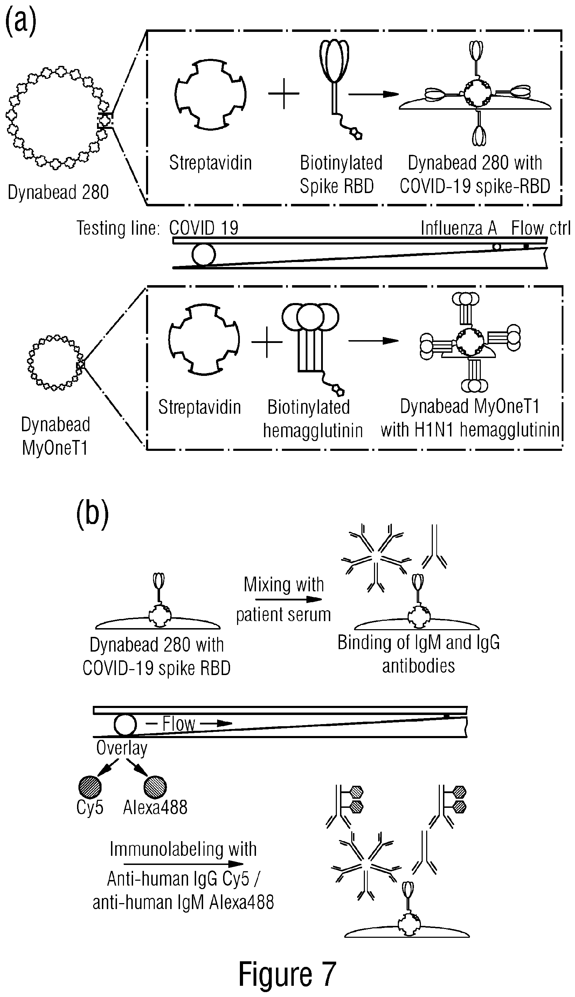

Figure 7 represents a conceptual drawing illustrating the on-chip multiplexing concept to simultaneously detect IgG and IgM antibodies and disease multiplexing. Figure 7(a) Top: functionalization of 2.8 µm streptavidin coated nanoparticles with the receptor binding domain (RBD) of the spike protein. The entire trimeric protein with only one biotin moiety is drawn for simplification purposes. Middle: Schematic illustrating the on-chip disease multiplexing principle by using the two different sizes of nanoparticles. Bottom: The smaller nanoparticles beads are similarly functionalized with a biotinylated hemagglutinin protein Figure 7(b) shows Top and bottom: Schematic representation of the color multiplexing principle for simultaneous detection of IgG and IgM antibodies Middle: Cross-sectional sketch showing trapped nanoparticles and 0.9 µm flow control calibration beads.

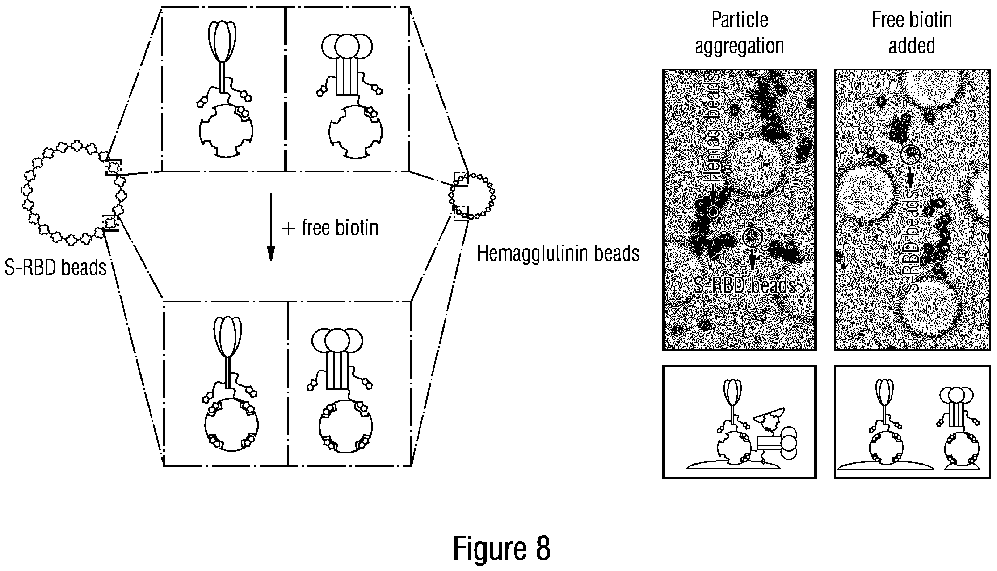

Figure 8 shows the principle towards disease multiplexing: saturating biotin-binding pockets of streptavidin. (left) Schematic illustrating the influence of free-biotin added during the blocking step on the available biotin binding sites. (right) 40x microscopy images of the 2.8 µm nanoparticle trapping linetrapping line, with no free-biotin added (left) and free biotin added (right).

Thus, a novel and innovative approach towards multiplexed antibody and disease testing is disclosed by using a unique 3D nanofluidic device. Proof of principle is obtained by showing that calibration-grade particles can be size-dependently immobilized. Their size can be accurately inferred from their trapping position. This principle was used to concentrate and trap S-RBD-functionalized beads in an IgG Covid-19 serological assay with a detection limit well within the physiologically relevant range. The test was further cross-validated with PCR-tested patient samples, showing a high degree of sensitivity and specificity.

Moreover, on-bead color multiplexing has shown the potential to simultaneously monitor the presence of both IgG and IgM antibodies in human sera on a single particle for future time-dependent antibody studies. Additionally, the versatility and applicability of the 3D nanofluidic device is shown by detecting anti-S-RBD (SARS-CoV-2) and anti-hemagglutinin (Influenza A) antibodies in control samples using distinct bead sizes.

For other embodiments of the present invention, the beads can be functionalized with a variety of biotinylated antigen targets, as there are multiple highly specific antibody targets of human immune system against SARS-CoV-2, such as the nucleocapsid (N) protein. Moreover, as the majority of current vaccines rather focus on the spike protein, functionalizing distinct bead sizes with these respective proteins, enables the rapid determination of the origin of patient's COVID-19 immunity. This is of the utmost importance to when a large fraction of the population is vaccinated to provide rapid large-scale information about vaccine efficacy.

### Methods

### Gray-scale e-beam lithography.

PMMA was spin-coated on a Si-wafer to achieve an average film thickness of 4 µm. The resist was exposed with different dose squares of by using Raith EBPG 5000+ electron beam system operated at a 100 kV acceleration voltage. The PMMA was developed in pure methyl isobutyl ketone (MIBK). The remaining resist depth of the exposed areas was measured and plotted against the respective exposure dose to obtain the contrast curve. A Monte-Carlo simulation of the interaction between the incoming electron beam and resist material to obtain the point-spread function (PSF) at a given penetration depth. The nanofluidic device was designed in a GDSII format, where each layer was correlated with a different height to achieve the desired 3D topography in the trapping region and the connecting wedge. The contrast curve, PSF and nanofluidic design were used to perform proximity effect correction before e-beam exposure, after which the resist was developed as previously described.

Nanofluidic master fabrication and nano-imprint lithography. The surface of the glass was activated with an oxygen plasma. Immediately after that, an adhesion promotor was spin-coated on the activated surface. Afterwards, a UV crosslinkable polymer was pipetted directly on the surface of the developed nanofluidic e-beam structure, on top of which the glass wafer was added. Subsequently, the polymer was cured under UV-light. The nanofluidic master was put into an imprinting tool with a free-standing PMMA film on top of it, as well as an anti-adhesion coated silicon wafer and poly(amide)-PDMS-poly(amide) sandwich. Hereafter, the imprinting tool was heated above the glass transition temperature of the thermoplastic material and the nanofluidic structures were embossed. The imprinted film was activated by oxygen plasma and spin-coated with a Dextran solution in MilliQ. The film was then mechanically cut into separate devices and the particle protecting dextran layer was removed by submerging the chips in MilliQ water.

### Poly(vinyl alcohol) (PVA) functionalization and bonding.

The surfaces of the 1 mm patterned film and a 200 µm unpatterned film were activated by UV/O-activation. Subsequently, a PVA solution in PBS was spin-coated. To remove any excess PVA and aid film homogeneity, the surface of the PMMA nanofluidic chip was spin-washed with MilliQ at. Afterward, the patterned and unpatterned films were aligned and bonded at elevated pressure and temperature 750 N and 45°C for 1 min.

### Trapping of calibration grade fluorescent polystyrene particles

A five-particle mixture in PBS was made with calibration grade fluorescent polystyrene particles of the following sizes: 0.9 µm, 1.02 µm, 1.744 µm, 2.12 µm, 3.16 µm. A droplet was put onto the inflow section of the nanofluidic device and after the liquid reached the end of the capillary pump region, a droplet of PBS was put at the outflow to prevent premature drying of the sample while fluorescently characterizing the trapping position of the particles. A custom python script was used to analyze the average trapping positions of the particles and corresponding standard deviation. The particle sizes specified by the supplier were used to fit a linear regression curve to the particle trapping position. The mean particle size and coefficient of variation were recalculated from the linear regression fit.

### Preparation of 2.8 µm and 1 µm nanoparticles and with spike-RBD and hemagglutinin.

*2.8 µm nanoparticles.* The beads were added to PBS and left on a magnet to allow for proper magnetic isolation of the beads. The washed beads were mixed together with the biotinylated receptor-binding domain (RBD) spike protein. Subsequently, the beads were washed in PBS. For the COVID-19 only experiments, the particles were blocked with Superblock PBS, whereas for the influenza multiplexing experiments, 0.9 mg/mL free biotin was added during the blocking step to saturate all remaining open streptavidin binding pockets.

*1 µm nanoparticles.* The beads were washed as described in the previous section and were labeled with biotinylated Influenza A H1N1 Hemagglutinin. The beads were subsequently blocked in Superblock PBS containing 0.9 mg/mL free biotin.

Serological immunoassay - limit-of-detection and test validation. The proof-of-principle of on-bead and on-chip immunoassay was performed by using a chimeric antibody and diluting it in COVID-19 negative human serum. To detect the binding of the antibodies to the antigen-coated beads, the washed particles were incubated with Cy5 donkey anti-human IgG and subsequently washed. To monitor the flow and trapping functionality of the device, 0.9 µm non-magnetic nanoparticles were added to the suspension. A droplet of the suspension was then loaded onto the 3D nanofluidic chip and after the liquid made its way to the end of the capillary pump, a droplet of PBS was pipetted on the outflow region to halt the further flow of the fluid. The fluorescent signal from the particles was then investigated by fluorescent microscopy in the relevant trapping region and quantified by using a custom Python image analysis script.

*Assay Validation.* To validate the assay, the same assay was performed with a 29-patient sample set, consisting of 19 IgG positive and 10 IgG negative patients. All of the IgG positive patients had a positive PCR test for COVID-19, whereas the IgG-negative patients had a negative PCR test. The highest fluorescent signal of the negative patient population was taken as a cut-off value. A higher signal was considered to be a positive result.

### Antibody and disease multiplexing.

*Antibody multiplexing.* 2.8 µm nanoparticles were labeled with the SARS-CoV-2 spike RBD as previously outlined. Subsequently, they were mixed and incubated with serum from a positive patient control with a high ELISA titer for IgG and IgM antibodies against the spike RBD as well as with a negative patient control, which was tested PCR negative for COVID-19. To allow for color multiplexed antibody detection, two secondary antibodies were added to the particle suspension: donkey anti-human IgM Alexa 488 and donkey anti-human IgG Cy5 and incubated with the washed particle suspension as described in the previous section. The device load and investigation as well as the associated analysis was also performed in the same fashion.

*Disease multiplexing.* As a proof-of-principle experiment to highlight the disease multiplexing capabilities of the 3D nanofluidic device, a particle suspension containing spike RBD functionalized 2.8 µm nanoparticles and hemagglutinin functionalized 1 µm nanoparticles particles were mixed with purified polyclonal rabbit anti-spike (and purified polyclonal rabbit anti-hemagglutinin. The beads were then immunostained with donkey anti-rabbit Cy3 after which the suspension was washed with PBS. 0.9 µm flow control beads were added before loading the particles into the nanofluidic device. The relevant trapping lines were investigated with fluorescence microscopy and the signal for each particle size was analyzed with a custom python script.

## Claims

1. A nanofluidic device for geometrical particle immobilization and trapping, preferably but not limited to, for rapid and multiplexed serological antibody detection, comprising:
a) a channel wall defining by its surrounding structure a channel having a trapping section of decreasing cross-sectional area as seen in a flow direction of a fluid comprising the particles to be trapped dependent on their geometry;
b) said trapping section being penetrated by a plurality of support columns; said columns supporting the channel wall from collapsing;
c) said plurality of column supports are formed as free-standing columns in the material of the channel wall.

2. The device according to claim 1, wherein the distance of adjacent support columns is chosen to be larger than the size of the smallest particles to be trapped.

3. The device according to claim 1 or 2, wherein the cross-sectional area of the trapping section is chosen to be at its inlet larger than the size of the biggest particles to be trapped and at its outlet smaller than the size of the smallest particles to be trapped.

4. The device according to any of the preceding claims wherein the channel and its trapping section are designed to convey a fluid by capillary force.

5. The device according to any of the preceding claims wherein a plurality of channels and respective trapping section are comprised.

6. The device according to any of the preceding claims wherein the material of the channel wall is a thermoplastic.

7. The device according to any of the preceding claims wherein the column supports are generated by replication methods of a negative master pattern copy into a free-standing substrate thereby generating a patterned substrate.

8. The device according to claim 7, wherein the channel and its trapping section are generated by UV/O-assisted bonding of the patterned and an unpatterned substrate.

9. The device, according to any of the preceding claims, wherein the support columns have a cross-sectional area in the range from 10 µm² to 2000 µm², preferably in the range from 20 µm² to 70 µm².

10. The device according to any of the preceding claims where the course of cross-sectional area is designed to separate a multiparticle mixture at distinct positions within the trapping section.

11. The device according to claim 10 where each trapping section is used to perform an on-bead immunoassay.

12. The device according to any of the preceding claims wherein multiple inflows are provided to perform distinct immunoassays for different antigen targets.

## Patentansprüche

1. Nanofluidische Vorrichtung für geometrisches Partikelimmobilisieren und -einfangen, vorzugsweise für, aber nicht beschränkt auf, schnelle und multiplexierte serologische Antikörperdetektion, umfassend:
a) eine Kanalwand, die durch ihre umgebende Konstruktion einen Kanal definiert, der einen Einfangabschnitt mit einer abnehmenden Querschnittsfläche in Strömungsrichtung eines Fluids gesehen aufweist, das die in Abhängigkeit von ihrer Geometrie einzufangenden Partikel umfasst;
b) wobei der Einfangabschnitt von einer Vielzahl von Stützsäulen durchsetzt ist, wobei die Säulen die Kanalwand gegen Zusammenfallen abstützen;
c) wobei die Vielzahl von Säulenstützen als freistehende Säulen in dem Material der Kanalwand ausgebildet ist.

2. Vorrichtung nach Anspruch 1, wobei der Abstand benachbarter Stützsäulen größer als die Größe der kleinsten einzufangenden Partikel gewählt ist.

3. Vorrichtung nach Anspruch 1 oder 2, wobei die Querschnittsfläche des Einfangabschnitts an seinem Einlass größer als die Größe der größten einzufangenden Partikel und an seinem Auslass kleiner als die Größe der kleinsten einzufangenden Partikel gewählt ist.

4. Vorrichtung nach einem der vorhergehenden Ansprüche, wobei der Kanal und sein Einfangabschnitt zum Fördern eines Fluids durch Kapillarkraft ausgestaltet sind.

5. Vorrichtung nach einem der vorhergehenden Ansprüche, wobei eine Vielzahl von Kanälen und jeweiligem Einfangabschnitt umfasst ist.

6. Vorrichtung nach einem der vorhergehenden Ansprüche, wobei das Material der Kanalwand ein Thermoplast ist.

7. Vorrichtung nach einem der vorhergehenden Ansprüche, wobei die Säulenstützen durch Replikationsverfahren mit einer negativen Master-Musterkopie in ein freistehendes Substrat erzeugt sind, wodurch ein gemustertes Substrat erzeugt ist.

8. Vorrichtung nach Anspruch 7, wobei der Kanal und sein Einfangabschnitt durch UV/O-gestütztes Bonden des gemusterten und eines ungemusterten Substrats erzeugt sind.

9. Vorrichtung nach einem der vorhergehenden Ansprüche, wobei die Stützsäulen eine Querschnittsfläche im Bereich von 10 µm² bis 2000 µm², vorzugsweise im Bereich von 20 µm² bis 70 µm², aufweisen.

10. Vorrichtung nach einem der vorhergehenden Ansprüche, wobei der Verlauf der Querschnittsfläche derart ausgestaltet ist, dass ein Multipartikelgemisch an verschiedenen Positionen innerhalb des Einfangabschnitts getrennt wird.

11. Vorrichtung nach Anspruch 10, wobei jeder Einfangabschnitt zum Durchführen eines Bead-basierten Immunoassays verwendet wird.

12. Vorrichtung nach einem der vorhergehenden Ansprüche, wobei mehrere Zuflüsse zum Durchführen verschiedener Immunoassays für unterschiedliche Antigenziele bereitgestellt sind.

## Revendications

1. Dispositif nanofluidique destiné à l'immobilisation et au piégeage de particules géométriques, de préférence mais sans s'y limiter, destiné à la détection d'anticorps sérologique rapide et multiplexée, comprenant :
a) une paroi de canal définissant par sa structure environnante un canal présentant une section de piégeage de surface transversale décroissante telle qu'observée dans une direction d'écoulement d'un fluide comprenant les particules à piéger en fonction de leur géométrie ;
b) ladite section de piégeage étant pénétrée par une pluralité de colonnes de support ; lesdites colonnes supportant la paroi de canal pour l'empêcher de s'effondrer ;
c) ladite pluralité de colonnes de support sont formées en tant que colonnes autonomes dans le matériau de la paroi de canal.

2. Dispositif selon la revendication 1, dans lequel la distance de colonnes de support adjacentes est choisie pour être supérieure à la taille des plus petites particules à piéger.

3. Dispositif selon la revendication 1 ou 2, dans lequel la surface transversale de la section de piégeage est choisie pour être, au niveau de son entrée, supérieure à la taille des plus grosses particules à piéger et, au niveau de sa sortie, inférieure à la taille des plus petites particules à piéger.

4. Dispositif selon l'une quelconque des revendications précédentes, dans lequel le canal et sa section de piégeage sont conçus pour transporter un fluide par la force capillaire.

5. Dispositif selon l'une quelconque des revendications précédentes, dans lequel une pluralité de canaux et une section de piégeage respective sont compris.

6. Dispositif selon l'une quelconque des revendications précédentes, dans lequel le matériau de la paroi de canal est un thermoplastique.

7. Dispositif selon l'une quelconque des revendications précédentes, dans lequel les supports de colonne sont générés par des procédés de réplication d'une copie de modèle maître négatif dans un substrat autonome générant ainsi un substrat à motif.

8. Dispositif selon la revendication 7, dans lequel le canal et sa section de piégeage sont générés par liaison assistée par UV/O du substrat à motif et d'un substrat sans motif.

9. Dispositif, selon l'une quelconque des revendications précédentes, dans lequel les colonnes de support présentent une surface transversale dans la plage de 10 µm² à 2000 µm², de préférence dans la plage de 20 µm² à 70 µm².

10. Dispositif selon l'une quelconque des revendications précédentes, dans lequel le cours de la surface transversale est conçu pour séparer un mélange à particules multiples au niveau de positions distinctes au sein de la section de piégeage.

11. Dispositif selon la revendication 10, dans lequel chaque section de piégeage est utilisée pour réaliser un immunoessai sur particules.

12. Dispositif selon l'une quelconque des revendications précédentes, dans lequel de multiples flux entrants sont fournis pour réaliser des immunoessais distincts pour différentes cibles antigéniques.
